# EUROPEAN PATENT SPECIFICATION

(11) **EP 2 740 494 B1**
(45) Date of publication and mention of the grant of the patent: **28.11.2018**
(21) Application number: 12460087.5
(22) Date of filing: 10.12.2012
(51) Int. Cl.: A61L 2/00, B01J 19/12, A61L 2/12, H05B 6/80

(54) **Microwave sterilizer for liquids**
Mikrowellensterilisator für Flüssigkeiten
Stérilisateur micro-ondes pour liquides

(43) Date of publication of application: 11.06.2014
(73) Proprietor: Enbio Technology Sp. Z o.o., 81-198 Kosakowo (PL)
(72) Inventor: Krajczynski, Marek, 81 326 Gdynia (PL)
(74) Representative: Czabajski, Jacek

(56) References cited:
- US-A- 5 345 066
- US-B1- 6 579 501

## Description

The object of the invention is a microwave sterilizer for the sterilization of liquids, including all kinds of biological material in a liquid or semi-solid phase, especially microbiological culture media, or food samples, in particular in the laboratory.

Sterilization processes of liquid biological material are usually carried out in a flow, or in an autoclave-type closed container with the use of raised temperature in a gaseous medium or in an aqueous medium, where in the case of sterilization of biological material, steam autoclaves are primarily used because it is possible to precisely maintain the temperature regime, where in the case of water, the boiling point, crucial for biological material, is strictly defined and, moreover, can be reached at different temperatures, depending on the pressure inside the autoclave. At normal pressure, the boiling point of water is 100°C and reaching this point limits further temperature rise. The increase in the pressure inside the autoclave enables the increase in the temperature of the boiling point of water. The pressure inside the autoclave is usually adjustable by means of a pressure valve.

The total time required to carry out a sterilization process is the sum of the water bath heating time, the temperature adjustment time of water bath and biological material or other material being sterilised, the sterilization time, when a proper sterilization process takes place during which the set temperature should be maintained for a specified time, and the autoclave cooling time. In subsequent technical solutions of liquid sterilizers the aim is to reduce this total time, what is especially important in the laboratory, where during research work small samples of biological material must be sterilized a number of times.

In the solution known from US patent specification No. US 4,808,783, a sterilization method with the use of microwave energy is disclosed. According to this known solution, the method involves heating a product, where in one part of the microwave device the product is heated faster and in the other it is heated slower to a uniform predetermined temperature sufficient to sterilize the product without the loss of the product properties such as odour, taste or vitamin content.

In the solution known from US 6579501 B1 specification there are disclosed an apparatus were when the reaction liquid is heated the pressure in the interior of the pressure vessel will increase. Another known device for heating substances under the development of high pressures in a microwave field is known from US patent specification US 5,345,066.

Another solution known from US patent specification No. US 2008/0083749 discloses another solution of an apparatus and method for the dehydration and/or sterilization of organic materials. According to this known solution, the apparatus comprises microwave chamber, at least one microwave generator for irradiating material within the autoclave, and a vacuum system coupled to the microwave chamber to reduce the pressure below atmospheric pressure. In one embodiment the autoclave drum is rotatable and helical vanes are arranged inside.

Another known solution is disclosed in US patent specification No. US 5,108,701. The solution relates to a process of rapid sterilization of biological media. According to this known solution, a process of rapid sterilisation of biological media, such as gels or broths and their incubation as growth media to test the presence of microorganisms in tested samples, includes applying microwave radiation to such a medium contained in a microwave transmissive pressure container. Sterilization takes place by directing the microwave radiation through the container onto the medium in such amount that the temperature and pressure in the container are raised, and maintaining such parameters for a short time, usually less than ten minutes, is sufficient to sterilise the biological medium. In a preferred embodiment the microwave heating takes place for about five minutes, and the microwave heating of the medium takes place in a reaction chamber of an apparatus, where at the same time a plurality of containers with the biological medium can be present. The chamber is subjected to an oscillating motion, what additionally facilitates the maintenance of uniform parameters of the material sterilization process. After the sterilization process is complete the medium is ready for use.

Another known solution of a microwave steriliser is disclosed in US patent specification No. 4,406,860. According to this known solution, sterilization takes place in a non-metallic container with a metallic or non-metallic lid. The container is placed within a microwave heating chamber equipped with a valve for limiting the pressure within the enclosure during the process and preventing the entry of air during the process and during the cooling process.

The aim of the invention is to develop a portable steriliser unit, especially a laboratory sterilizer with a shortened total time of the sterilization process from the time of placing biological material in the sterilizer to the time of removing the sterilized material from the sterilizer. This aim is solved according to claim 1 and subsequent claims.

According to the invention, a microwave sterilizer for the sterilization of liquids, comprising a body containing a process chamber with an airtight lid. At least one magnetron which is the source of microwaves with an electrical power supply and cooling system is attached to the body. The body (2) and the process chamber mounted on the body form together a resonance chamber into which an antenna of at least one magnetron is inserted. The walls and bottom of the process chamber are made at least partially of material permeable to microwave radiation, and the process chamber is connected a pressure pneumatic system containing a system of pipes supplied with gas under pressure with a pressure and temperature adjustment and measuring unit and a microprocessor controller.

According to the invention the process chamber is further connected to a hydraulic water supply and discharge system for dispensing water in order to provide a water jacket inside the process chamber during a sterilization process for a vessel containing the medium being sterilized.

In a preferred embodiment according to the invention, antennae of two magnetrons are inserted into the resonance chamber.

The invention provides that antennae of magnetrons can be inserted into the resonance chamber in the area of the process chamber base.

The process chamber, according to the invention, can be equipped with a water level connector.

The walls and bottom of the process chamber, according to the invention, can be made at least partially of material permeable to microwave radiation, in particular of polytetrafluoroethylene.

However, the resonance chamber casing of the sterilizer is made of electromagnetically tight material, in particular of metal.

In the solution according to the invention, the process chamber lid is preferably made of electromagnetically tight material, in particular of metal.

For the connection of the openable process chamber lid with the side walls of the chamber a sealing means is provided, preferably in the form of a gasket which has a cross-sectional shape of the letter V, where the arms of the letter V of the seal are directed towards the interior of the process chamber.

A new solution of a microwave sterilizer is disclosed, in particular for the sterilization of liquid biological material. In the sterilization process overpressure is used to increase the boiling point threshold of the material. A solution of the sterilizer with the possibility of controlled pressure reduction with the use of a high-frequency switching valve is proposed. The device enables the change of dynamics of the rate of pressure reduction for each stage of the sterilization process separately. The term a stage of the sterilization process should be understood here as subsequent value of the changed temperature. This enables a rapid pressure reduction while avoiding sudden boiling which may result in undesirable boiling over of a medium, in particular a medium containing biological material.

Inside the process chamber a water jacket is used which has been experimentally proven to normalise the heating process conditions so that, within certain limits, regardless of the size of a vessel or the volume of a sterilized medium, the total volume of liquid in the process chamber is constant thus making it possible to obtain the same process parameters.

The object of the invention is presented in an embodiment in the accompanying drawings, where individual figures present:
- Fig. 1 -: a schematic section of a resonance chamber with a sterilizer process chamber,
- Fig. 2 -: a detail of a section of a lid seal,
- Fig. 3 -: a detail of a section of a resonance chamber and process chamber contact edge seal,
- Fig. 4 -: a view of a steriliser after removing side walls and a top wall,
- Fig. 5 -: a side view of a complete sterilizer,
- Fig. 6 -: a top view of the resonance chamber with the process chamber according to Fig. 1,
- Fig. 7 -: a view of a steriliser unit of a top wall,
- Fig. 8 -: a view of a steriliser unit of a front wall,
- Fig. 9 -: a view of a steriliser unit of a back wall.

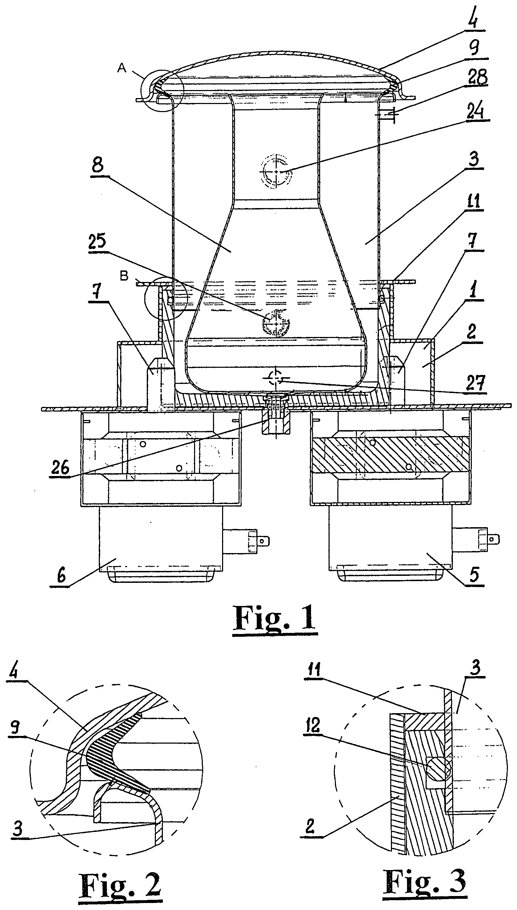

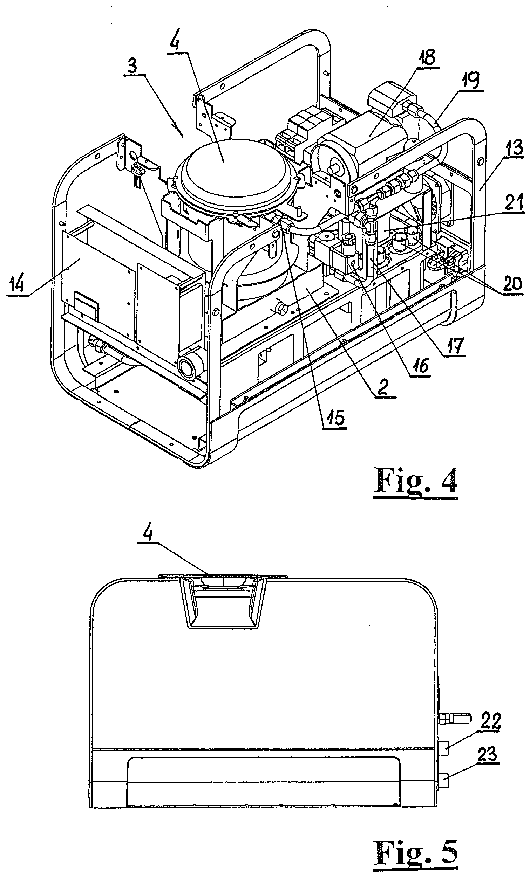

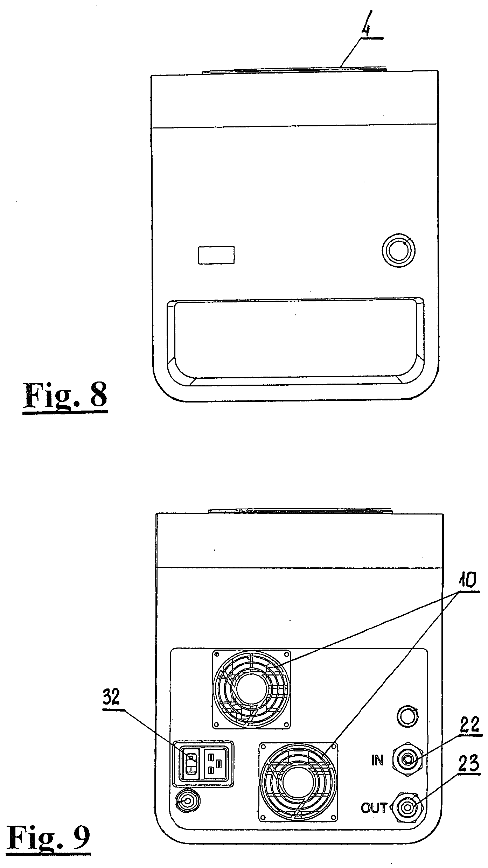

A general view of the microwave sterilizer after removing covering walls is shown in Fig. 4. However, Fig. 1 shows a schematic section of a resonance chamber 1, comprising a body 2 with a process chamber 3 partly arranged inside the resonance chamber 1. The resonance chamber 1 has in this embodiment the body 2 with the process chamber 3 closed with a lid 4. The process chamber 3 is arranged in the resonance chamber 1 of the body 2. Thus, the body 2, the process chamber 3 and the process chamber lid 4 together is the resonance chamber 1. This is clearly shown in Fig. 1. The same figure shows magnetrons 5,6 which are the source of microwaves being a working medium in the sterilization process of biological material. The magnetrons 5,6 are attached in this embodiment from the bottom to the resonance chamber 1 body 2. As shown in Fig. 1, antennae 7 of the magnetrons 5,6 are put into the interior of the resonance chamber 1. The magnetrons 5,6 contain an external power supply system and their own cooling system in the form of fans 10 shown in Fig. 9. Fig. 1 shows a vessel 8, schematically arranged inside the process chamber, with liquid biological material for sterilization.

The process chamber 3 is closed with the airtight lid 4. In this embodiment, a gasket 9 is used for sealing the contact of the lid and the process chamber. As shown in Fig. 2, the gasket 9 has a cross-sectional shape of the letter V, where the arms of the letter V are directed towards the interior of the process chamber 3.

The process chamber, as shown in the embodiment in Fig. 1, is mounted with its bottom part inside the resonance chamber 1. The resonance chamber 1 body 2 upper flange 11 surrounds the cylindrical process chamber 3 and the contact of the flange with the process chamber 3 wall is sealed by means of an peripheral seal 12. This is shown in detail in Fig. 3.

A view of the microwave sterilizer in the embodiment, after removing covering walls, is shown in Fig. 4. This figure shows a frame 13, in which all functional sterilizer units, according to the present invention, are fixed. The process chamber 3 with the lid 4 and the resonance chamber 1 body 2 are fixed in the frame 13. In addition, a controller 14 is fixed with a microprocessor system carrying out a sequence of changes in pressure and temperature parameters, leading to the sterilization of a sample in the vessel 8.

A pressure air pipe with an air nozzle 15 is provided to the process chamber 3. The sterilizer unit also contains a pressure valve 16 on a pipe 17 in an air pressure system. In this embodiment, the air pressure system is supplied by means of a pumping pipe 19 from its own compressor 18. The aim is to obtain the possibility of increasing and decreasing pressure inside the process chamber 3 for the effective control of the temperature of the boiling point of the liquid inside the vessel 8 put into the process chamber 3. In other embodiments, the steriliser can be supplied by an external air pressure source. In the sterilizer system, gas other than air can be used to increase pressure.

An important element of the microwave sterilizer, according to the present invention, is a hydraulic system, containing a water supply pipe with a supply connector 22 and a discharge pipe with a water discharge connector 23 to/from the process chamber 3. This is shown in Fig. 9, where grilles of cooling fans 10 of magnetrons 5,6 are also shown. In Fig. 1 inside the process chamber 3 a water supply nozzle 24 and a water level nozzle 25, as well as a water discharge nozzle 26 are shown. The process chamber 3 is also equipped with a temperature sensor 27 and an air nozzle 28.

The electrical system of magnetrons 5,6 contains known high-voltage capacitors 20 and transformers 21, as well as an electric supply system.

The process chamber is, thus, connected with a pressure pneumatic system containing the system of pipes supplied with air under pressure. Inlet and outlet of the pressure pneumatic system is arranged in the area of the resonance chamber in which the process chamber is arranged. The sterilizer is also equipped with a known pressure adjustment and measurement unit, as well as a temperature sensor in the process chamber and a controller with a microprocessor.

The accompanying figures shows that antennae 7 of two magnetrons 5,6 are put into the resonance chamber 1. In other embodiments the number of magnetrons used may be different. In this embodiment, the figures show that antennae 7 of magnetrons 5,6 are put into the interior of the resonance chamber 1 in the area of the process chamber 3 base. Other arrangements of antennae 7 of magnetrons 5,6 in other embodiments are not excluded.

The walls and bottom of the process chamber 3 are made in the bottom part of material permeable to microwave radiation, in this embodiment they are made of polytetrafluoroethylene. This is important especially in the area of the anticipated interaction of microwaves on the sterilized material, where the interior of the process chamber 3 is part of the whole resonance chamber. The walls of the process chamber 3 can be made in the upper part of material impermeable to microwave radiation, for example of metal. This is shown in Fig. 1.

However, the whole outer casing of the resonance chamber 1 body 2 of the sterilizer in this embodiment is made of metal, namely of electromagnetically tight material.

Also the process chamber 3 lid 4 is made of electromagnetically tight material, in this embodiment of metal. For the connection of the openable process chamber 3 lid 4 with the side walls of the chamber 3 in this embodiment a gasket 9 is used. This is shown in Fig. 1 and Fig. 2. In this embodiment the process chamber 3 is connected with the resonance chamber 1 separably. Fig. 1 and Fig. 3 show an o-ring-type sealing means 12, sealing the contact of the process chamber 3 wall and the resonance chamber 1 upper flange 11.

Fig. 5 shows a side view of the sterilizer unit with a water supply connector 22 and a water discharge connector 23.

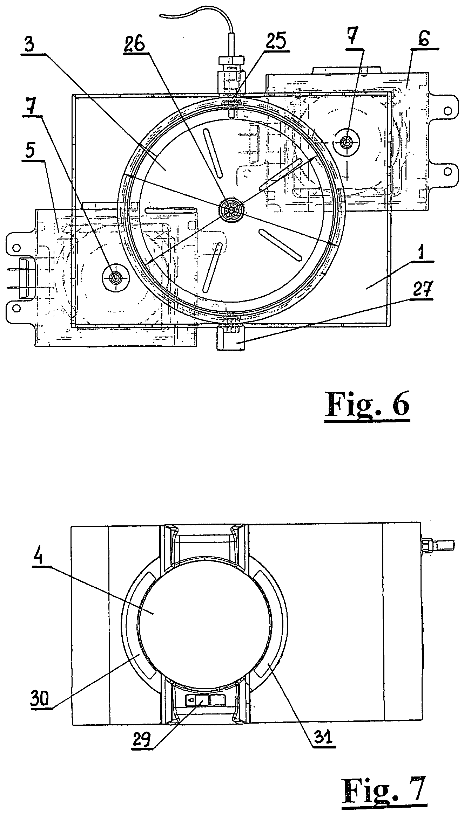

Fig. 6 shows a schematic view of the resonance chamber 1 and the process chamber 3 arranged therein. The process chamber 3 here is part of the resonance chamber 1. In addition, magnetrons 5,6 fixed under the resonance chamber 1 and antennae 7 of the magnetrons 5,6 put into the interior of the resonance chamber 1 are shown.

The upper lid of the sterilizer unit is shown in a view in Fig. 7. This figure illustrates the process chamber 3 lid 4. An outer element of the lid 4 lock 29 is shown which position of opening and closing is controlled by a controller 14 and depends on the completion of a full cycle of the sterilization process in the process chamber 3 saved in the microprocessor of the controller 14. In the state open or closed to LED light arcs 30,31 at the same time a signal of the change in the colour of the light is provided depending on the current state of closing or opening the lid 4.

Fig. 8 and Fig. 9 show a view of the front wall and a view of the back wall of the sterilizer. Fig. 9 shows a power socket 32, grilles of cooling fans 10 of magnetrons 5,6 and supply 22 and discharge connectors 23 of water supplying the process chamber 3.

A vessel 8 with liquid material being sterilized is placed in the process chamber 3. The lid 4 is closed and light arcs 30, 31 indicate the correctness of closing. Then, in an automatic cycle a specified quantity of water is dispensed to the process chamber 3. During the sterilization process the vessel 8 with the medium being sterilized, is in a water jacket inside the process chamber 3. Then, a specified quantity of air is pumped into enabling the pushing out of the excess water through a water level nozzle 25 quickly.

This makes that in the process chamber 3 water in the water jacket has always constant and the same level, regardless of the size of the vessel 8 with material being sterilized.

Air is still pumped into the process chamber 3 until the predetermined pressure is obtained which will prevent boiling of the material in the vessel during heating.

Then, microwave heating is turned on. Microwaves magnetrons 5,6 at the same time heat water in the jacket in the process chamber 3 and the material for sterilization in the vessel 8. After reaching the set sterilization temperature, microwave heating turns off and the process of controlled reduction of pressure in the process chamber 3 begins. At a strictly defined point of this process a specified quantity of cold water is pumped into the process chamber. When pressure is reduced to zero, the device indicates the end of the process. Then, the process chamber 3 lid 4 can be opened and the vessel 8 containing the sterilized material can be removed.

### The list of designations in the figures

- 1.: Resonance chamber.
- 2.: Resonance chamber body.
- 3.: Process chamber.
- 4.: Process chamber lid.
- 5.: Magnetron.
- 6.: Magnetron.
- 7.: Antenna.
- 8.: Vessel with material for sterilisation.
- 9.: Gasket.
- 10.: Fan.
- 11.: Resonance chamber upper flange.
- 12.: Peripheral seal.
- 13.: Frame.
- 14.: Controller.
- 15.: Air nozzle.
- 16.: Air pressure valve.
- 17.: Outlet air pipe.
- 18.: Compressor.
- 19.: Air pipe.
- 20.: High-voltage capacitor.
- 21.: Transformer.
- 22.: Water supply connector.
- 23.: Water discharge connector.
- 24.: Water supply nozzle.
- 25.: Water level nozzle.
- 26.: Water discharge nozzle.
- 27.: Temperature sensor.
- 28.: Air nozzle.
- 29.: Lid lock.
- 30.: LED light arc.
- 31.: LED light arc.
- 32.: Power socket.

## Claims

1. A microwave sterilizer for the sterilization of liquids, comprising a body (2) containing a process chamber (3) with an airtight lid (4), and to which at least one magnetron (5,6) which is the source of microwaves with an electrical power supply and cooling system is attached, wherein the body (2) and the process chamber (3) mounted on the body (2) form together (3) a resonance chamber (1) into which an antenna (7) of at least one magnetron (5) is inserted, where the walls and bottom of the process chamber (3) are made at least partially of material permeable to microwave radiation, and wherein the process chamber is connected to a pressure pneumatic system containing a system of pipes supplied with gas under pressure with a pressure and temperature adjustment and measuring unit and a microprocessor controller, **characterised in that** the process chamber (3) is further connected to a hydraulic water supply and discharge system for dispensing water in order to provide a water jacket inside the process chamber (3) during a sterilization process for a vessel (8) containing the medium being sterilized.

2. The sterilizer, according to claim 1, **characterised in that** the walls and bottom of the process chamber (3) are made of polytetrafluoroethylene.

3. The sterilizer, according to claim 1, **characterised in that** antennae (7) of two magnetrons (5,6) are inserted into the resonance chamber (1).

4. The sterilizer, according to claim 2, **characterised in that** antennae (7) of magnetrons (5,6) are inserted into the resonance chamber (1) in the area of the process chamber (3) bottom.

5. The sterilizer, according to claim 1, **characterised in that** the process chamber (3) is equipped with a water level nozzle (25).

6. The sterilizer, according to claim 1, **characterised in that** the resonance chamber (1) casing is made of electromagnetically tight material, in particular of metal.

7. The sterilizer, according to claim 1 or 6, **characterised in that** the process chamber (3) lid (4) is made of electromagnetically tight material, in particular of metal.

8. The sterilizer, according to claim 7, **characterised in that** the connection of the process chamber (3) lid (4) with the side walls of the process chamber (3) contains a sealing means.

9. The sterilizer, according to claim 7, **characterised in that** the sealing mean is a gasket (9) which has a cross-sectional shape of the letter V, where the arms of the letter V are directed towards the interior of the process chamber (3).

## Patentansprüche

1. Mikrowellensterilisator für Flüssigkeiten, bestehend aus dem Körper (2), in dem sich die Prozesskammer (3) mit einem dichten Deckel (4) befindet, und an dem mindestens ein Magnetron (5, 6) als Mikrowellenquelle mit elektrischer Stromquelle zur Versorgung und Kühlung befestigt ist, wobei der Körper (2) und die auf dem Körper, zusammen mit der Resonanzkammer, befestigte Prozesskammer (3) mit der hydraulischen Wasserzufuhr und Wasserableitung verbunden ist, wobei sich in der Resonanzkammer (1) eine Antenne (7) mindestens eines Magnetrons (5) befindet und die Wände und der Boden der Prozesskammer (3) mindestens teilweise aus einem für Mikrowellen durchlässigen Material ausgeführt sind, und wobei die Prozesskammer an ein Luftdrucksystem mit einem System von mit unter Druck stehendem Gas versorgten Rohren mit Druck- und Temperaturmesssystem und Mikroprozessorsteuerung angeschlossen ist, **dadurch gekennzeichnet, dass** die Prozesskammer (3) auch mit dem hydraulischen Versorgungs- und Wasserverteilungssystem zur Wasserdosierung zur Versorgung des Wassermantels in der Prozesskammer (3) bei der Sterilisierung zum Gefäß (8) mit der sterilisierten Flüssigkeit verbunden ist.

2. Mikrowellensterilisator nach Anspruch 1, **dadurch gekennzeichnet, dass** die Wände und der Boden der Prozesskammer (3) aus Polytetrafluorethylen ausgeführt sind.

3. Mikrowellensterilisator nach Anspruch 1, **dadurch gekennzeichnet, dass** im Innenraum der Resonanzkammer (1) Antennen (7) zweier Magnetrone (5,6) eingeführt sind.

4. Mikrowellensterilisator nach Anspruch 2, **dadurch gekennzeichnet, dass** die Antennen (7) der Magnetrone (5,6) im Innenraum der Resonanzkammer (1) im Bodenbereich der Prozesskammer (3) eingeführt sind.

5. Mikrowellensterilisator nach Anspruch 1, **dadurch gekennzeichnet, dass** die Prozesskammer (3) mit Wasserniveau-Düsen (25) ausgestattet ist.

6. Mikrowellensterilisator nach Anspruch 1, **dadurch gekennzeichnet, dass** der Körper der Resonanzkammer (1) aus einem elektromagnetisch dichten Kunststoff, insbesondere aus Metall ausgeführt ist.

7. Mikrowellensterilisator nach Anspruch 1 oder 4, **dadurch gekennzeichnet, dass** der Deckel (4) der Prozesskammer (3) aus einem elektromagnetisch dichten Kunststoff, insbesondere aus Metall ausgeführt ist.

8. Mikrowellensterilisator nach Anspruch 6, **dadurch gekennzeichnet, dass** die Verbindung des Deckels (4) der Prozesskammer (3) mit den Seitenwänden dieser Prozesskammer (3) ein Dichtungsmittel enthält.

9. Mikrowellensterilisator nach Anspruch 7, **dadurch gekennzeichnet, dass** das Dichtungsmittel eine Dichtung (9) in V-Form im Querschnitt ist, wobei die Arme des Buchstabens V in Richtung des Innenraumes der Prozesskammer (3) zeigen.

## Revendications

1. Stérilisateur micro-ondes pour liquides, contenant un corps (2) dans lequel se trouve une chambre de processus (3) avec un couvercle étanche (4), et auquel est fixé au moins un magnétron (5,6) étant source de micro-ondes avec le système électrique de son alimentation et son refroidissement où le corps (2) et la chambre de processus (3) sont montés sur le corps (2) avec la chambre de résonance (1) est lié au système hydraulique d'approvisionnement et d'évacuation de l'eau, et dans la chambre de résonance (1) il y a une antenne (7) d'au moins un magnétron (5), et les parois et le fond de la chambre de processus (3) sont faits au moins partiellement de matériau perméable au rayonnement micro-ondes, et où la chambre de processus est liée au système pneumatique sous pression comprenant un système de tuyaux alimentés en gaz sous pression, avec une unité de mesure de la pression et de la température et un contrôleur à microprocesseur, **caractérisé en ce que** la chambre de processus (3) est connectée également au système hydraulique d'alimentation et de distribution de l'eau pour le dosage de l'eau alimantant la chemise d'eau à l'intérieur de la chambre de processus (3) pendant le processus de stérilisation au récipient (8) contenant un agent à stériliser.

2. Stérilisateur selon la revendication 1, **caractérisé en ce que** les parois et le fond de la chambre de processus (3) sont faits de polytétrafluoroéthylène.

3. Stérilisateur selon la revendication 1, **caractérisé en ce qu'à** l'intérieur de la chambre de résonance (1) sont insérées les antennes (7) de deux magnétrons (5,6).

4. Stérilisateur selon la revendication 2, **caractérisé en ce que** les antennes (7) des magnétrons (5,6) sont insérées à l'intérieur de la chambre de résonance (1) dans la zone de fond de la chambre de processus (3).

5. Stérilisateur selon la revendication 1, **caractérisé en ce que** la chambre de processus (3) est équipée de la buse de niveau d'eau (25).

6. Stérilisateur selon la revendication 1, **caractérisé en ce que** le boîtier de la chambre de résonance (1) est fait de matériau étanche électromagnétiquement, en particulier de métal.

7. Stérilisateur selon la revendication 1 ou 4, **caractérisé en ce que** le couvercle (4) de la chambre de processus (3) est fait de matériau étanche électromagnétiquement, en particulier de métal.

8. Stérilisateur selon la revendication 6, **caractérisé en ce que,** la connexion du couvercle (4) de la chambre de processus (3) aux parois latérales de cette chambre de processus (3) contient un produit d'étanchéité.

9. Stérilisateur selon la revendication 7, **caractérisé en ce que** le moyen d'étanchéité est joint d'étanchéité (9) sous forme de la lettre V en coupe transversale, les bras de la lettre V sont orientés vers l'intérieur de la chambre de processus (3).
